(19) Europäisches Patentamt
European Patent Office
Office européen des brevets

(11) EP 2 046 839 B2

(12) NEUE EUROPÄISCHE PATENTSCHRIFT
Nach dem Einspruchsverfahren

(45) Veröffentlichungstag und Bekanntmachung des Hinweises auf die Entscheidung über den Einspruch:
**10.04.2019 Patentblatt 2019/15**

(45) Hinweis auf die Patenterteilung:
**12.05.2010 Patentblatt 2010/19**

(21) Anmeldenummer: **07787356.0**

(22) Anmeldetag: **11.07.2007**

(51) Int Cl.:
***C08F 2/10*** *(2006.01)* ***C08F 20/04*** *(2006.01)*

(86) Internationale Anmeldenummer:
**PCT/EP2007/057082**

(87) Internationale Veröffentlichungsnummer:
**WO 2008/009599 (24.01.2008 Gazette 2008/04)**

# (54) VERFAHREN ZUR HERSTELLUNG WASSERABSORBIERENDER POLYMERPARTIKEL MIT HOHER PERMEABILITÄT DURCH POLYMERISATION VON TROPFEN EINER MONOMERLÖSUNG

METHOD FOR PRODUCING WATER-ABSORBENT POLYMER PARTICLES WITH A HIGHER PERMEABILITY BY POLYMERISING DROPLETS OF A MONOMER SOLUTION

PROCÉDÉ PERMETTANT LA PRODUCTION DE PARTICULES POLYMÈRES ABSORBANT L'EAU À PERMÉABILITÉ ÉLEVÉE GRÂCE À LA POLYMÉRISATION DE GOUTTELLETTES D'UNE SOLUTION MONOMÈRE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **19.07.2006 EP 06117489**

(43) Veröffentlichungstag der Anmeldung:
**15.04.2009 Patentblatt 2009/16**

(73) Patentinhaber: **BASF SE**
**67056 Ludwigshafen am Rhein (DE)**

(72) Erfinder:
- **STUEVEN, Uwe**
  **65812 Bad Soden (DE)**
- **WEISMANTEL, Matthias**
  **63637 Jossgrund (DE)**
- **HEIDE, Wilfried**
  **67251 Freinsheim (DE)**
- **KRÜGER, Marco**
  **68161 Mannheim (DE)**
- **SEIDL, Volker**
  **68199 Mannheim (DE)**
- **BLEI, Stefan**
  **68163 Mannheim (DE)**
- **LÖSCH, Dennis**
  **67122 Altrip (DE)**
- **FUNK, Rüdiger**
  **65527 Niedernhausen (DE)**
- **HILLEBRECHT, Annemarie**
  **36100 Petersberg (DE)**

(74) Vertreter: **BASF IP Association**
**BASF SE**
**G-FLP-C006**
**67056 Ludwigshafen (DE)**

(56) Entgegenhaltungen:
**EP-A- 0 521 355**
**EP-A2- 0 349 241**
**EP-A2- 1 029 886**
**EP-A2- 1 433 526**
**WO-A-96/40427**
**WO-A1-01/41818**
**WO-A1-2004/069293**
**WO-A1-2005/027987**
**WO-A1-2005/030810**
**WO-A1-2005/037875**
**WO-A1-2005/073260**
**WO-A1-2006/014031**
**WO-A2-2006/058685**
**DE-A1- 10 340 253**
**DE-A1-102004 024 437**
**JP-A- H1 180 248**
**JP-A- H09 255 704**
**US-A- 5 073 612**
**US-A- 5 669 894**
**US-A1- 2006 014 031**
**US-A1- 2006 252 899**
**US-B1- 6 620 889**



Printed by Jouve, 75001 PARIS (FR)

**Beschreibung**

Die vorliegende Erfindung betrifft ein Verfahren zur Herstellung wasserabsorbierender Polymerpartikel mit hoher Permeabilität durch Polymerisation von Tropfen einer Monomerlösung, enthaltend Säuregruppen tragende Monomere, in einer die Tropfen umgebenden Gasphase, wobei die die Monomerlösung mehrwertige Kationen enthält und die Polymerpartikel einem mittleren Durchmesser von mindestens 150 μm aufweisen.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Wasserabsorbierende Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Die Eigenschaften der wasserabsorbierenden Polymere können über den Vernetzungsgrad eingestellt werden. Mit steigendem Vernetzungsgrad steigt die Gelfestigkeit und sinkt die Absorptionskapazität. Dies bedeutet, dass mit steigender Absorption unter Druck (AUL) die Zentrifugenretentionskapazität (CRC) abnimmt (zu sehr hohen Vernetzungsgraden nimmt auch die Absorption unter Druck wieder ab).

Zur Verbesserung der Anwendungseigenschaften, wie beispielsweise Flüssigkeitsweiterleitung im gequollenen Gelbett (SFC) in der Windel und Absorption unter Druck (AUL), werden wasserabsorbierende Polymerpartikel im allgemeinen nachvernetzt. Dadurch steigt nur der Vernetzungsgrad der Partikeloberfläche, wodurch die Absorption unter Druck (AUL) und die Zentrifugenretentionskapazität (CRC) zumindest teilweise entkoppelt werden können. Diese Nachvernetzung kann in wässrig Gelphase durchgeführt werden. Vorzugsweise werden aber gemahlene und abgesiebte Polymerpartikel (Grundpolymer) an der Oberfläche mit einem Nachvernetzer beschichtet, thermisch nachvernetzt und getrocknet. Dazu geeignete Vernetzer sind Verbindungen, die mindestens zwei Gruppen enthalten, die mit den Carboxylatgruppen des hydrophilen Polymeren kovalente Bindungen bilden können.

Durch Sprühpolymerisation konnten die Verfahrensschritte Polymerisation und Trocknung zusammengefasst werden. Zusätzlich konnte die Partikelgröße durch geeignete Verfahrensführung in gewissen Grenzen eingestellt werden.

Die Herstellung wasserabsorbierender Polymerpartikel durch Polymerisation von Tropfen einer Monomerlösung wird beispielsweise in EP-A0 348 180, WO 96/440427, US 5,269,980, DE-A 103 14 466, DE-A 103 40 253 und DE-A 10 2004 024 437 sowie den älteren deutschen Anmeldungen mit den Aktenzeichen 10 2005 002 412.2 und 10 2006 001 596.7 beschrieben.

DE-A 10 2004 042 946, DE-A 10 2004 042 948 und DE-A 10 2004 042 955 sowie die ältere deutsche Anmeldung mit dem Aktenzeichen 10 2005 019 398.6 beschreiben die Herstellung von Verdickern durch Sprühpolymerisation.

Aufgabe der vorliegenden Erfindung war die Bereitstellung eines Verfahrens zur Herstellung wasserabsorbierender Polymerpartikel mit hoher Permeabilität, d.h. einer hohen Flüssigkeitsweiterleitung durch das gequollene Gelbett.

Gelöst wurde die Aufgabe durch ein Verfahren zur Herstellung wasserabsorbierender Polymerpartikel durch Polymerisation von Tropfen einer Monomerlösung, enthaltend

a) mindestens ein Säuregruppen tragendes ethylenisch ungesättigtes Monomer,

b) mindestens einen Vernetzer,

c) mindestens einen Initiator,

d) Wasser,

in einer die Tropfen umgebenden Gasphase, dadurch gekennzeichnet, dass der Vernetzer b) mindestens zwei radikalisch polymerisierbare Gruppen aufweist, die Monomerlösung von 0,001 bis 0,25 Gew.-%, bezogen auf Monomer a), mindestens dreiwertige Kationen enthält und die Polymerpartikel einem mittleren Durchmesser von mindestens 150 μm aufweisen.

Die gemäß dem erfindungsgemäßen Verfahren erhältlichen wasserabsorbierenden Polymerpartikel weisen eine Permeabilität (SFC) von typischerweise mindestens 5 x $10^{-7}$ $cm^3s/g$, vorzugsweise mindestens 15 x $10^{-7}$ $cm^3s/g$, bevorzugt mindestens 35 x $10^{-7}$ $cm^3s/g$, besonders bevorzugt mindestens 50 x $10^{-7}$ $cm^3s/g$, ganz besonders bevorzugt mindestens 120 x $10^{-7}$ $cm^3s/g$, auf. Die Permeabilität (SFC) der wasserabsorbierenden Polymerpartikel beträgt üblicherweise weniger als 500 x $10^{-7}$ $cm^3s/g$.

Die gemäß dem erfindungsgemäßen Verfahren erhältlichen wasserabsorbierenden Polymerpartikel weisen eine Zentrifugenretentionskapazität (CRC) von typischerweise mindestens 10 g/g, vorzugsweise mindestens 15 g/g, bevorzugt mindestens 20 g/g, besonders bevorzugt mindestens 25 g/g, ganz besonders bevorzugt mindestens 30 g/g, auf. Die Zentrifugenretentionskapazität (CRC) der wasserabsorbierenden Polymerpartikel beträgt üblicherweise weniger als 50 g/g.

Geeignete mehrwertige Kationen sind dreiwertige Kationen, wie die Kationen von Aluminium, Eisen, Chrom, Seltenerden und Mangan, vierwertige Kationen, wie die Kationen von Titan und Zirkonium. Dreiwertige Kationen sind bevorzugt. Als Gegenion sind Chlorid, Bromid, Sulfat, Hydrogensulfat, Carbonat, Hydrogencarbonat, Nitrat, Phosphat, Hydragenphosphat, Dihydrogenphosphat und Carboxylat, wie Acetat und Lactat, möglich. Aluminiumsalze, wie Aluminiumsulfat ist bevorzugt.

Die Menge an mehrwertigem Kation beträgt 0,001 bis 0,25 Gew.-%, besonders bevorzugt 0,01 bis 0,2 Gew.-%, ganz besonders bevorzugt 0,05 bis 0,15 Gew.-%, jeweils bezogen auf das Monomer a).

Die Menge an mehrwerigen Kation ist dabei so zu wählen, dass in der Monomerlösung weder Trübungen noch Ausfällungen auftreten.

Der mittlere Durchmesser der Polymerpartikel beträgt vorzugsweise mindestens 200 µm, besonders bevorzugt von 250 bis 600 µm, ganz besonders von 300 bis 500 µm, wobei der Partikeldurchmesser durch Lichtstreuung bestimmt werden kann und den volumengemittelten mittleren Durchmesser bedeutet. 90% der Polymerpartikel weisen einen Durchmesser von vorzugsweise 100 bis 800 µm, besonders bevorzugt von 150 bis 700 µm, ganz besonders bevorzugt von 200 bis 600 µm, auf.

Der Sauerstoffgehalt der Gasphase beträgt vorzugsweise 0,001 bis 0,15 Vol.-%, besonders bevorzugt 0,002 bis 0,1 Vol.-%, ganz besonders bevorzugt 0,005 bis 0,05 Vol.-%.

Die Gasphase enthält neben Sauerstoff vorzugsweise nur inerte Gase, d. h. Gase, die unter Reaktionsbedingungen nicht in die Polymerisation eingreifen, beispielsweise Stickstoff und/oder Wasserdampf.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 50 g/100 g Wasser, und haben vorzugsweise mindestens je eine Säuregruppe.

Die Konzentration der Monomeren a) in der Monomerlösung beträgt üblicherweise 2 bis 80 Gew.-%, vorzugsweise 5 bis 70 Gew.-%, besonders bevorzugt 10 bis 60 Gew.-%.

Geeignete Monomere a) sind beispielsweise ethylenisch ungesättigte Carbonsäuren, wie Acrylsäure, Methacrylsäure, Maleinsäure, Fumarsäure und Itaconsäure. Besonders bevorzugte Monomere sind Acrylsäure und Methacrylsäure. Ganz besonders bevorzugt ist Acrylsäure.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Säuregruppen der Monomere a) sind üblicherweise teilweise neutralisiert, vorzugsweise zu 25 bis 85 mol-%, bevorzugt zu 50 bis 80 mol-%, besonders bevorzugt 60 bis 75 mol-%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumcarbonat oder Natriumhydrogencarbonat sowie deren Mischungen. üblicherweise wird die Neutralisation durch Einmischung des Neutralisationsmittels als wässrige Lösung, als Schmelze, oder bevorzugt auch als Feststoff erreicht. Beispielsweise kann Natriumhydroxid mit einem Wasseranteil deutlich unter 50 Gew.-% als wachsartige Masse mit einem Schmelzpunkt oberhalb 23°C vorliegen. In diesem Fall ist eine Dosierung als Stückgut oder Schmelze bei erhöhter Temperatur möglich.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ) und/oder Tocopherole.

Unter Tocopherol werden Verbindungen der folgenden Formel verstanden

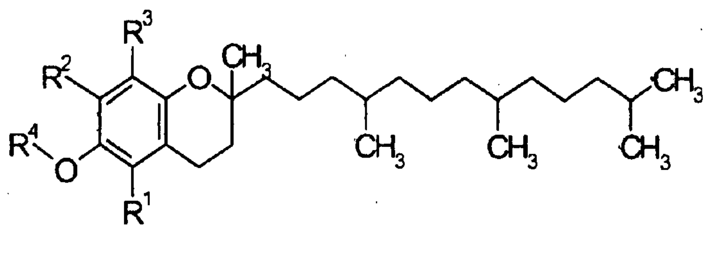

wobei $R^1$ Wasserstoff oder Methyl, $R^2$ Wasserstoff oder Methyl, $R^3$ Wasserstoff oder Methyl und $R^4$ Wasserstoff oder ein Säurerest mit 1 bis 20 Kohlenstoffatomen bedeutet.

Bevorzugte Reste für $R^4$ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit $R^1 = R^2 = R^3$ = Methyl, insbesondere racemisches alpha-Tocopherol. $R^1$ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindesten 10 Gew.-ppm, besonders bevorzugt mindesten 30 Gew.-ppm, insbesondere um 50 Gew.-ppm, Hydrochinonhalbether, jeweils bezogen auf Acrylsäure, wobei Acrylsäuresalze als Acrylsäure mit berücksichtigt werden. Beispielsweise kann zur Herstellung der Monomerlösung eine Acrylsäure mit einem entsprechenden Gehalt an Hydrochinonhalbether verwendet werden.

Die Vernetzer b) sind Verbindungen mit mindestens zwei radikalisch polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Allylmethacrylat, Trimethylolpropantriacrylat, Triallylamin, Tetraallyloxyethan, wie in EP-A 0 530 438 beschrieben, Di- und Triacrylate, wie in EP-A 0 547 847, EP-A 0 559 476, EP-A 0 632 068, WO 93/21237, WO 03/104299, WO 03/104300, WO 03/104301 und in DE-A 103 31 450 beschrieben, gemischte Acrylate, die neben Acrylatgruppen weitere ethylenisch ungesättigte Gruppen enthalten, wie in DE-A 103 314 56 und DE-A 103 55 401 beschrieben, oder Vernetzermischungen, wie beispielsweise in DE-A 195 43 368, DE-A 196 46 484, WO 90/15830 und WO 02/32962 beschrieben.

Geeignete Vernetzer b) sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A 0 343 427 beschrieben sind. Weiterhin geeignete Vernetzer b) sind Pentaerythritoldi- Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1.000 aufweist.

Besonders vorteilhafte Vernetzer b) sind jedoch Di- und Triacrylate des 3- bis 15-fach ethoxylierten Glyzerins, des 3- bis 15-fach ethoxylierten Trimethylolpropans, des 3- bis 15-fach ethoxylierten Trimethylolethans, insbesondere Di- und Triacrylate des 2- bis 6- fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des 40-fach ethoxylierten Glyzerins, Trimethylolethans oder Trimethylolpropans.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine wie sie beispielsweise in WO 03/104301 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5- fach ethoxylierten und/oder propoxylierten Glyzerins. Am meisten bevorzugt sind die Triacrylate des 3- bis 5-fach ethoxylierten und/oder propoxylierten Glyzerins. Diese zeichnen sich durch besonders niedrige Restgehalte (typischerweise unter 10 Gew.-ppm) im wasserabsorbierenden Polymer aus und die wässrigen Extrakte der damit hergestellten wasserabsorbierenden Polymere weisen eine fast unveränderte Oberflächenspannung (typischerweise mindestens 0,068 N/m) im Vergleich zu Wasser gleicher Temperatur auf.

Die Monomerlösung enthält typischerweise mindestens 0,2 Gew.-%, vorzugsweise mindestens 0,6 Gew.-%, bevorzugt mindestens 0,8 Gew.-%, besonders bevorzugt mindestens 1,5 Gew.-%, ganz besonders bevorzugt mindestens 3,0 Gew.-%, Vernetzer b), jeweils bezogen auf Monomer a).

Als Initiatoren c) können sämtliche unter den Polymerisationsbedingungen in Radikale zerfallende Verbindungen eingesetzt werden, beispielsweise Peroxide, Hydroperoxide, Wasserstoffperoxid, Persulfate, Azoverbindungen und die sogenannten Redoxinitiatoren. Bevorzugt ist der Einsatz von wasserlöslichen Initiatoren. In.manchen Fällen ist es vorteilhaft, Mischungen verschiedener Initiatoren zu verwenden, beispielsweise Mischungen aus Wasserstoffperoxid und Natrium- oder Kaliumperoxodisulfat. Mischungen aus Wasserstoffperoxid und Natriumperoxodisulfat können in jedem beliebigen Verhältnis verwendet werden.

Besonders bevorzugte Initiatoren c) sind Azoinitiatoren, wie 2,2'-Azobis[2-(2-imidazolin-2-yl)propan]dihydrochlorid und 2,2'-Azobis[2-(5-methyl-2-imidazolin-2-yl)propan]dihydrochlorid, und Photoinitiatoren, wie 2-Hydroxy-2-methylpropiophenon und 1-[4-(2-Hydroxyethoxy)-phenyl]-2-hydroxy-2-methyl-1-propan-1-on, Redoxinitiatoren, wie Natriumpersulfat/ Hydroxymethylsulfinsäure, Ammoniumperoxodisulfat/Hydroxymethylsulfinsäure, Wasserstoffperoxid/Hydroxymethylsulfinsäure, Natriumpersulfat/Ascorbinsäure, Ammoniumperoxodisulfat/Ascorbinsäure und Wasserstoffperoxid/Ascorbinsäure, Photoinitiatoren, wie 1-[4-(2-Hydroxyethoxy)-phenyl]-2-hydroxy-2-methyl-1-propan-1-on, sowie deren Mischungen.

Die Initiatoren werden in üblichen Mengen eingesetzt, beispielsweise in Mengen von 0,001 bis 5 Gew.-%, vorzugsweise 0,01 bis 1 Gew.-%, bezogen auf die Monomeren a).

Die bevorzugten Polymerisationsinhibitoren benötigen für eine optimale Wirkung gelösten Sauerstoff. Daher kann die Monomerlösung vor der Polymerisation durch Inertisierung, d. h. Durchströmen mit einem inerten Gas, vorzugsweise Stickstoff, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, gesenkt.

Die Polymerisationsinhibitoren können auch durch Absorption, beispielsweise an Aktivkohle, entfernt werden.

Die Monomerlösung wird zur Polymerisation in der Gasphase vertropft.

Die Polymerisation in den Monomerlösungstropfen findet in homogener Phase statt, wobei die durch das suspendierte anorganische Salz bedingten Inhomogenitäten unberücksichtigt bleiben. Dies bedeutet, dass die Monomerlösung homogen ist und dass die Monomerlösung auch während der Polymerisation homogen bleibt. Das Polymer darf während der Polymerisation quellen, aber nicht ausfallen und eine zweite Phase im Tropfen bilden. Ansonsten würden in jedem Tropfen mehrere Polymerkeime entstehen, die während der Trocknung Agglomerate sehr kleiner Primärpartikel bilden. Ziel des erfindungsgemäßen Verfahrens ist die Herstellung jeweils eines Primärpartikels pro Tropfen. Daher sind die Monomeren a) und die Vernetzer b) so auszuwählen, dass das entstehende Polymer in der wässrigen Phase des Tropfens quellbar ist.

Daher wird das erfindungsgemäße Verfahren vorzugsweise in Abwesenheit hydrophober inerter Lösungsmittel durchgeführt. Hydrophobe, inerte Lösungsmittel sind praktisch alle mit Wasser nicht mischbaren Flüssigkeiten, die nicht in die Polymerisation eingreifen, d.h. keine polymerisierbaren Gruppen enthalten. Mit Wasser nicht mischbar bedeutet, dass die Löslichkeit der hydrophoben Lösungsmittel in Wasser weniger als 5 g/100 g, vorzugsweise weniger als 1 g/100 g, besonders bevorzugt weniger als 0,5 g/100 g, beträgt.

Bei der Vertropfung wird eine Monomerlösung unter Ausbildung von Tropfen in die Gasphase dosiert. Die Vertropfung der Monomerlösung kann beispielsweise mittels einer Vertropferplatte durchgeführt werden.

Eine Vertropferplatte ist eine Platte mit mindestens einer Bohrung, wobei die Flüssigkeit von oben durch die Bohrung tritt. Die Vertropferplatte bzw. die Flüssigkeit kann in Schwingungen versetzt werden, wodurch an der Unterseite der Vertropferplatte je Bohrung eine idealerweise monodisperse Tropfenkette erzeugt wird.

Die Anzahl und die Größe der Bohrungen werden gemäß der gewünschten Kapazität und Tropfengröße ausgewählt. Der Tropfendurchmesser beträgt dabei üblicherweise das 1,9fache des Durchmessers der Bohrung. Wichtig ist hierbei, dass die zu vertropfende Flüssigkeit nicht zu schnell durch die Bohrung tritt bzw. der Druckverlust über die Bohrung nicht zu groß ist. Ansonsten wird die Flüssigkeit nicht vertropft, sondern der Flüssigkeitsstrahl wird infolge der hohen kinetischen Energie zerrissen (versprüht). Der Vertropfer wird im Strömungsbereich des laminaren Strahlzerfalls betrieben, d.h. die Reynoldszahl bezogen auf den Durchsatz pro Bohrung und den Bohrungsdurchmesser ist vorzugsweise kleiner als 2.000, bevorzugt kleiner 1.000, besonders bevorzugt kleiner 500, ganz besonders bevorzugt kleiner 100. Der Druckverlust über die Bohrung beträgt vorzugsweise weniger als 2,5 bar, besonders bevorzugt weniger als 1,5 bar, ganz besonders bevorzugt weniger als 1 bar.

Die Vertropferplatte weist üblicherweise mindestens eine, vorzugsweise mindestens 10, besonders bevorzugt mindestens 50, und üblicherweise bis zu 10.000, vorzugsweise bis zu 5.000, besonders bevorzugt bis zu 1.000, Bohrungen auf, wobei die Bohrungen üblicherweise gleichmäßig über die Vertropferplatte verteilt sind, vorzugsweise in der sogenannten Dreiecksteilung, d.h. jeweils drei Bohrungen bilden die Ecken eines gleichseitigen Dreiecks.

Der Durchmesser der Bohrungen wird an die gewünschte Tropfengröße angepasst.

Es kann vorteilhaft sein die Vertropferplatte auf eine Trägerplatte aufzulegen, wobei die Trägerplatte ebenfalls Bohrungen aufweist. Dabei weisen die Bohrungen der Trägerplatte einen größeren Durchmesser auf als die Bohrungen der Vertropferplatte auf und sind so angeordnet, dass sich unter jeder Bohrung der Vertropferplatte eine mit ihr konzentrische Bohrung der Trägerplatte befindet. Diese Anordnung ermöglicht einen schnellen Wechsel der Vertropferplatte, beispielsweise um Tropfen einer anderen Größe zu erzeugen.

Die Vertropfung kann aber auch mittels pneumatischer Ziehdüsen, Rotation, Zerschneiden eines Strahls oder schnell ansteuerbarer Mikroventildüsen durchgeführt werden.

In einer pneumatischen Ziehdüse wird ein Flüssigkeitsstrahl zusammen mit einem Gasstrom durch eine Blende beschleunigt. Über die Gasmenge kann der Durchmesser des Flüssigkeitsstrahls und damit der Tropfendurchmesser beeinflusst werden.

Bei der Vertropfung durch Rotation tritt die Flüssigkeit durch die Öffnungen einer rotierenden Scheibe. Durch die auf die Flüssigkeit wirkende Fliehkraft werden Tropfen definierter Größe abgerissen. Die Rotationsvertropfung wird beispielsweise in DE-A 4308842 und US 6338438 beschrieben.

Der austretende Flüssigkeitsstrahl kann aber auch mittels eines rotierenden Messers in definierte Segmente zerschnitten werden. Jedes Segment bildet anschließend einen Tropfen.

Bei Verwendung von Mikroventildüsen werden direkt Tropfen mit definiertem Flüssigkeitsvolumen erzeugt.

Bevorzugt strömt die Gasphase als Trägergas durch den Reaktionsraum. Dabei kann das Trägergas im Gleichstrom oder im Gegenstrom zu den frei fallenden Tropfen der Monomerlösung durch den Reaktionsraum geführt werden, bevorzugt im Gleichstrom. Vorzugsweise wird das Trägergas nach einem Durchgang zumindest teilweise, bevorzugt zu mindestens 50%, besonders bevorzugt zu mindestens 75%, als Kreisgas in den Reaktionsraum zurückgeführt. Üblicherweise wird eine Teilmenge des Trägergases nach jedem Durchgang ausgeschleust, vorzugsweise bis zu 10%, besonders bevorzugt bis zu 3%, ganz besonders bevorzugt bis zu 1%.

Die Polymerisation wird vorzugsweise in einer laminaren Gasströmung durchgeführt. Eine laminare Gasströmung ist eine Gasströmung, bei der sich die einzelnen Schichten der Strömung nicht vermischen, sondern parallel bewegen. Ein Maß für die Strömungsverhältnisse ist die Reynolds-Zahl (Re). Unterhalb einer kritischen Reynolds-Zahl ($Re_{krit}$) von 2300 ist die Gasströmung laminar. Die Reynolds-Zahl der laminaren Gasströmung beträgt vorzugsweise weniger als 2000, besonders bevorzugt weniger als 1500, ganz besonders bevorzugt weniger als 1000. Der untere Grenzfall der laminaren Inertgasströmung ist eine ruhende Inertgasatmosphäre (Re = 0), d.h., es wird nicht kontinuierlich Inertgas eingespeist.

Die Gasgeschwindigkeit wird vorzugsweise so eingestellt, dass die Strömung im Reaktor gerichtet ist, beispielsweise liegen keine der allgemeinen Strömungsrichtung entgegengesetzte Konvektionswirbel vor, und beträgt beispielsweise 0,1 bis 2 m/s, vorzugsweise 0,5 bis 1,8 m/s, bevorzugt 1 bis 1,5 m/s.

Das Trägergas wird zweckmäßigerweise vor dem Reaktor auf die Reaktionstemperatur vorgewärmt.

Die Reaktionstemperatur beträgt bei der thermisch induzierten Polymerisation vorzugsweise 70 bis 250°C, besonders bevorzugt 100 bis 220°C, ganz besonders bevorzugt 120 bis 200°C.

Die Reaktion kann im Überdruck oder im Unterdruck durchgeführt werden, ein Unterdruck von bis zu 100 mbar gegenüber dem Umgebungsdruck ist bevorzugt.

Das Reaktionsabgas, d.h. das der Reaktionsraum verlassende Trägergas, kann beispielsweise in einem Wärmeaustauscher abgekühlt werden. Dabei kondensieren Wasser und nicht umgesetztes Monomer a). Danach kann das Reaktionsabgas zumindest teilweise wieder aufgewärmt und als Kreisgas in den Reaktor zurückgeführt werden. Ein Teil des Reaktionsabgases kann ausgeschleust und durch frisches Trägergas ersetzt werden, wobei im Reaktionsabgas enthaltenes Wasser und nicht umgesetzte Monomere a) abgetrennt und rückgeführt werden können.

Besonders bevorzugt ist ein Wärmeverbund, das heißt, ein Teil der Abwärme beim Abkühlen des Abgases wird zum Aufwärmen des Kreisgases verwendet.

Die Reaktoren können begleitbeheizt werden. Die Begleitheizung wird dabei so eingestellt, dass die Wandtemperatur mindestens 5°C oberhalb der Reaktorinnentemperatur liegt und die Kondensation an den Reaktorwänden zuverlässig vermieden wird.

Das Reaktionsprodukt kann dem Reaktor in üblicher Weise entnommen werden, beispielsweise am Boden über eine Förderschnecke, und gegebenenfalls bis zur gewünschten Restfeuchte und zum gewünschten Restmonomerengehalt getrocknet werden.

Selbstverständlich können die Polymerpartikel anschließend zur weiteren Verbesserung der Eigenschaften nachvernetzt werden.

Geeignete Nachvernetzer sind Verbindungen, die mindestens zwei Gruppen enthalten, die mit den Carboxylatgruppen des Hydrogels kovalente Bindungen bilden können. Geeignete Verbindungen sind beispielsweise Alkoxysiliylverbindungen, Polyaziridine, Polyamine, Polyamidoamine, Di- oder Polyglycidylverbindungen, wie in EP-A 0 083 022, EP-A 0 543 303 und EP-A 0 937 736 beschrieben, di- oder polyfunktionelle Alkohole, wie in DE-C 33 14 019, DE-C 35 23 617 und EP-A 0 450 922 beschrieben, oder β-Hydroxyalkylamide, wie in DE-A 102 04 938 und US 6,239,230 beschrieben.

Des weiteren sind in DE-A 40 20 780 zyklische Karbonate, in DE-A 198 07 502 2-Oxazolidon und dessen Derivate, wie 2-Hydroxyethyl-2-oxazolidon, in DE-A 198 07 992 Bis- und Poly-2-oxazolidinone, in DE-A 198 54 573 2-Oxotetrahydro-1,3-oxazin und dessen Derivate, in DE-A 198 54 574 N-Acyl-2-Oxazolidone, in DE-A 102 04 937 zyklische Harnstoffe, in DE-A 103 34 584 bizyklische Amidacetale, in EP-A 1 199 327 Oxetane und zyklische Harnstoffe und in WO 03/031482 Morpholin-2,3-dion und dessen Derivate als geeignete Nachvernetzer beschrieben.

Das erfindungsgemäße Verfahren ermöglicht die Herstellung wasserabsorbierender Polymerpartikel mit einer hohen Permeabilität (SFC) und einer hohen Zentrifugenretentionskapazität (CRC) erhalten werden. Für diese Eigenschaftskombination war bislang ein zusätzlicher Nachvernetzungsschritt zwingend erforderlich.

Die wasserabsorbierenden Polymerpartikel werden mittels der nachfolgend beschriebenen Testmethoden geprüft.

**Methoden**

Die Messungen sollten, wenn nicht anders angegeben, bei einer Umgebungstemperatur von 23 $\pm$ 2°C und einer relativen Luftfeuchte von 50 $\pm$ 10 % durchgeführt werden. Die wasserabsorbierenden Polymere werden vor der Messung gut durchmischt.

**Flüssigkeitsweiterleitung (SFC Saline Flow Conductivity)**

Die Flüssigkeitsweiterleitung einer gequollenen Gelschicht unter Druckbelastung von 0,3 psi (2070 Pa) wird, wie in EP-A 0 640 330 beschrieben, als Gel-Layer-Permeability einer gequollenen Gelschicht aus superabsorbierendem Polymer bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem

Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP-A-0 640 330. Der Durchfluss wird automatisch erfasst.

Die Flüssigkeitsweiterleitung SFC) wird wie folgt berechnet:

$$SFC\ [cm^3s/g] = (Fg(t{=}0) x L0)/(d x A x WP),$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, L0 die Dicke der Gelschicht in cm, d die Dichte der NaCl-Lösung in $g/cm^3$, A die Fläche der Gelschicht in $cm^2$ und WP der hydrostatische Druck über der Gelschicht in $dyn/cm^2$ darstellt.

**Zentrifugenretentionskapazität (CRC Centrifuge Retention Capacity)**

Die Zentrifugenretentionskapazität der wasserabsorbierenden Polymerpartikel wird gemäß der von der EDANA (European Disposables and Nonwovens Association) empfohlenen Testmethode Nr. 441.2-02 "Centrifuge retention capacity" bestimmt.

Die angegebenen Werte für die Zentrifugenretentionskapazität beziehen sich auf die wasserfreien wasserabsorbierenden Polymerpartikel, d.h. die gemessenen Werte wurden entsprechend dem Wassergehalt der wasserabsorbierenden Polymerpartikel vor der Messung korrigiert. Der Wassergehalt der wasserabsorbierende Polymerpartikel wird gemäß der von der EDANA (European Disposables and Nonwovens Association) empfohlenen Testmethode Nr. 430.2-02 "Moisture content" bestimmt.

Die EDANA-Testmethoden sind beispielsweise erhältlich bei der European Disposables and Nonwovens Association, Avenue Eugène Plasky 157, B-1030 Brüssel,Belgien.

**Beispiele**

14,6 kg Natriumacrylat (37,5 gew.-%ige Lösung in Wasser) und 1,4 kg Acrylsäure wurden mit 28,0 g 15-fach ethoxiliertem Trimethylolpropantriacrylat und 0 bis 55,9g Aluminiumsulfat gemischt. Die Lösung wurde in einen erwärmten, mit Stickstoffatmosphäre gefüllten Vertropfungsturm vertropft (180°C, 12m Höhe, 2m Breite, Gasgeschwindigkeit 0,1 m/s im Gleichstrom). Die Dosiergeschwindigkeit betrug 16 kg/h. Die Vertropferplatte wies 37 Bohrungen ä 170 µm auf. Der Durchmesser der Vertropferplatte betrug 65 mm. Der Initiator wurde kurz vor dem Vertropfer über einen Venturi-Mischer in die Monomerlösung dosiert. Als Initiator wurde eine 15 gew.-%ige Lösung von 2,2'-Azobis[2-(2-imidazolin-2-yl)propan]dihydrochlorid in Wasser verwendet. Die Dosiergeschwindigkeit der Initiatorlösung betrug 0,224 kg/h. Die Gasaustrittstemperatur aus dem Vertropfungsturm betrug 130°C. Der mittlere Teilchendurchmesser der erhaltenen Polymerpartikel betrug 270 µm.

Anschließend wurden die erhaltenen wasserabsorbierenden Polymerpartikel analysiert. Die Ergebnisse sind in Tabelle 1 zusammengefasst:

Tab. 1: Einfluss des mehrwertigen Kations

| Beispiel | Aluminiumsulfatmenge | Aluminiumsulfatgehalt*) | CRC [g/g] | SFC [$10^{-7}$ $cm^3s/g$] |
|---|---|---|---|---|
| 1 | 0,0 g | ohne | 39,2 | 4 |
| 2 | 28,0 g | 0,50 Gew.-% | 41,2 | 7 |
| 3 | 41,9 g | 0,75 Gew.-% | 40,4 | 12 |
| 4 | 55,9 g | 1,00 Gew.-% | 39,3 | 19 |
| *) bezogen auf Acrylsäure | | | | |

**Patentansprüche**

1. Verfahren zur Herstellung wasserabsorbierender Polymerpartikel durch Polymerisation von Tropfen einer Monomerlösung, enthaltend

   a) mindestens ein Säuregruppen tragendes ethylenisch ungesättigtes Monomer,

b) mindestens eines Vernetzers,
c) mindestens einen Initiator,
d) Wasser,

in einer die Tropfen umgebenden Gasphase, **dadurch gekennzeichnet, dass** der Vernetzer b) mindestens zwei radikalisch polymerisierbare Gruppen aufweist, die Monomerlösung von 0,001 bis 0,25 Gew.-%, bezogen auf Monomer a), mindestens dreiwertige Kationen enthält und die Polymerpartikel einem mittleren Durchmesser von mindestens 150 μm aufweisen.

**2.** Verfahren gemäß Anspruch 1, **dadurch gekennzeichnet, dass** das mindestens dreiwertige Kation ein Aluminiumkation ist.

**3.** Verfahren gemäß Anspruch 1 oder 2, **dadurch gekennzeichnet, dass** Monomer a) zu mindestens 50 mol-% Acrylsäure ist.

**4.** Verfahren gemäß einem der Ansprüche 1 bis 3, **dadurch gekennzeichnet, dass** die Polymerpartikel einen mittleren Durchmesser von mindestens 200 μm aufweisen.

**5.** Verfahren gemäß einem der Ansprüche 1 bis 4, **dadurch gekennzeichnet, dass** mindestens 90 Gew.-% der Polymerpartikel einen Durchmesser von 100 bis 800 μm aufweisen.

**6.** Verfahren gemäß einem der Ansprüche 1 bis 5, **dadurch gekennzeichnet, dass** ein Trägergas durch den Reaktionsraum strömt.

**7.** Verfahren gemäß Anspruch 6, **dadurch gekennzeichnet, dass** das den Reaktionsraum verlassende Trägergas nach einem Durchgang zumindest teilweise rückgeführt wird.

**8.** Verfahren gemäß einem der Ansprüche 1 bis 7, **dadurch gekennzeichnet**, der Sauerstoffgehalt des Trägergases von 0,001 bis 0,15 Vol.-% beträgt.

**9.** Verfahren gemäß einem der Ansprüche 1 bis 8, **dadurch gekennzeichnet, dass** die erhaltenen Polymerpartikel in mindestens einem weiteren Verfahrensschritt getrocknet und/oder nachvernetzt werden.

**10.** Wasserabsorbierende Polymerpartikel, herstellbar gemäß einem der Ansprüche 1 bis 9.

## Claims

**1.** A process for preparing water-absorbing polymer beads by polymerizing droplets of a monomer solution comprising

a) at least one ethylenically unsaturated monomer bearing acid groups,
b) at least one crosslinker,
c) at least one initiator,
d) water,

in a gas phase surrounding the droplets, wherein the crosslinker b) has at least two free-radically polymerizable groups, the monomer solution comprises from 0.001 to 0.25% by weight, based on monomer a), of at least trivalent cations and the polymer beads have a mean diameter of at least 150 μm.

**2.** The process according to claim 1, wherein the at least trivalent cation is an aluminum cation.

**3.** The process according to claim 1 or 2, wherein monomer a) is acrylic acid to an extent of at least 50 mol%.

**4.** The process according to any of claims 1 to 3, wherein the polymer beads have a mean diameter of at least 200 μm.

**5.** The process according to any of claims 1 to 4, wherein at least 90% by weight of the polymer beads have a diameter of from 100 to 800 μm.

**6.** The process according to any of claims 1 to 5, wherein a carrier gas flows through the reaction chamber.

**7.** The process according to claim 6, wherein the carrier gas leaving the reaction chamber is recycled at least partly after one pass.

**8.** The process according to any of claims 1 to 7, wherein the oxygen content of the carrier gas is from 0.001 to 0.15% by volume.

**9.** The process according to any of claims 1 to 8, wherein the resulting polymer beads are dried and/or postcrosslinked in at least one further process step.

**10.** Water-absorbing polymer beads preparable according to any of claims 1 to 9.

**Revendications**

**1.** Procédé de fabrication de particules polymères absorbant l'eau par polymérisation de gouttes d'une solution de monomères qui contient

a) au moins un monomère éthyléniquement insaturé portant des groupes acides,
b) au moins un agent de réticulation,
c) au moins un initiateur,
d) de l'eau,

dans une phase gazeuse entourant les gouttes, **caractérisé en ce que** l'agent de réticulation b) comporte au moins deux groupes polymérisables par voie radicalaire, **en ce que** la solution de monomères contient 0,001 à 0,25 % en poids, par rapport au monomère a), de cations au moins trivalents et **en ce que** les particules polymères présentent un diamètre moyen d'au moins 150 μm.

**2.** Procédé selon la revendication 1, **caractérisé en ce que** le cation au moins trivalent est un cation aluminium.

**3.** Procédé selon la revendication 1 ou 2, **caractérisé en ce que** le monomère a) est de l'acide acrylique à au moins 50 % en moles.

**4.** Procédé selon l'une quelconque des revendications 1 à 3, **caractérisé en ce que** les particules polymères présentent un diamètre moyen d'au moins 200 μm.

**5.** Procédé selon l'une quelconque des revendications 1 à 4, **caractérisé en ce qu'**au moins 90 % en poids des particules polymères présentent un diamètre de 100 à 800 μm.

**6.** Procédé selon l'une quelconque des revendications 1 à 5, **caractérisé en ce qu'**un gaz vecteur traverse la chambre de réaction.

**7.** Procédé selon la revendication 6, **caractérisé en ce que** le gaz vecteur qui quitte la chambre de réaction est recyclé au moins en partie après un passage.

**8.** Procédé selon l'une quelconque des revendications 1 à 7, **caractérisé en ce que** la teneur en oxygène du gaz vecteur est de 0,001 à 0,15 % en volume.

**9.** Procédé selon l'une quelconque des revendications 1 à 8, **caractérisé en ce que** les particules polymères obtenues sont séchées et/ou post-réticulées en au moins une étape de procédé supplémentaire.

**10.** Particules polymères absorbant l'eau, pouvant être fabriquées selon l'une quelconque des revendications 1 à 9.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 348180 A0
- WO 96440427 A
- US 5269980 A
- DE 10314466 A
- DE 10340253 A
- DE 102004024437 A
- DE 102005002412
- DE 102006001596
- DE 102004042946 A
- DE 102004042948 A
- DE 102004042955 A
- DE 102005019398
- EP 0530438 A
- EP 0547847 A
- EP 0559476 A
- EP 0632068 A
- WO 9321237 A
- WO 03104299 A
- WO 03104300 A
- WO 03104301 A
- DE 10331450 A
- DE 10331456 A
- DE 10355401 A
- DE 19543368 A
- DE 19646484 A
- WO 9015830 A
- WO 0232962 A
- EP 0343427 A
- DE 4308842 A
- US 6338438 B
- EP 0083022 A
- EP 0543303 A
- EP 0937736 A
- DE 3314019 C
- DE 3523617 C
- EP 0450922 A
- DE 10204938 A
- US 6239230 B
- DE 4020780 A
- DE 198075022 A
- DE 19807992 A
- DE 198545732 A
- DE 19854574 A
- DE 10204937 A
- DE 10334584 A
- EP 1199327 A
- WO 03031482 A
- EP 0640330 A


### In der Beschreibung aufgeführte Nicht-Patentliteratur


- **F.L. BUCHHOLZ ; A.T. GRAHAM.** Modern Superabsorbent Polymer Technology. Wiley-VCH, 1998, 71-103